# EUROPEAN PATENT APPLICATION

(11) **EP 4 834 515 A2**
(43) Date of publication of application: **04.11.2026**
(21) Application number: 26175979.9
(22) Date of filing: 30.04.2026
(51) Int. Cl.: B23K 9/32, A62B 18/00, B08B 15/04

(54) **PORTABLE SYSTEM TO CONTROL FUME EXTRACTION**

(30) Priority: 30.04.2025 US 202563797577 P; 29.04.2026 US 202619662098
(71) Applicant: Illinois Tool Works Inc., Glenview IL 60025 (US)
(72) Inventor: DYER, Karena, Glenview, 60025 (US); KOPAC III, Jordan, Glenview, 60025 (US); SCHNEIDER, Joseph C., Glenview, 60025 (US); SOMMERS, Eric, Glenview, 60025 (US)
(74) Representative: HGF

(57) **Abstract**

A fume extraction system is designed for metal working and other applications. The fume extraction system includes an air inlet arranged proximate to a nozzle of a welding tool; a body-worn suction device configured to draw in air from the air inlet, wherein the suction device further includes: a motor driving a fan to create suction that draws in the air from the air inlet; a filter to receive the air from the air inlet; and an air outlet to expel the filtered air.

## Description

### RELATED APPLICATIONS

The present application claims the benefit of U.S. Provisional Patent Application Serial No. 63/797,577, filed April 30, 2025, entitled "PORTABLE SYSTEMS AND METHODS TO CONTROL FUME EXTRACTION." The entirety of U.S. Provisional Patent Application Serial No. 63/797,577 is expressly incorporated herein by reference.

### FIELD OF THE DISCLOSURE

This disclosure relates generally to fume extraction and, more particularly, to portable systems and methods to control fume extraction.

### BACKGROUND

A wide range of industrial, commercial, hobby and other applications result in airborne components that can be removed with proper extraction and filtering. Metal working operations, for example, range from cutting, welding, soldering, assembly, and other processes that may generate smoke and fumes. In smaller shops it may be convenient simply to open ambient air passages or to use suction or discharge air from fans to maintain air spaces relatively clear. In other applications, enclosed and/or cart-type fume extraction systems are used. In industrial settings, more complex fixed systems may be employed for extracting fumes from specific works cells, metal working locations, and so forth. In other settings, such as machine shops, woodworking shops, worksites where cutting, sanding and other operations are performed, dust, fumes, particulate and other types of airborne components may be generated that it may be desirable to collect and extract from work areas and controlled spaces.

A number of systems have been developed for fume extraction, and a certain number of these are currently in use. In general, these use suction air to draw fumes and smoke from the immediate vicinity of the metal working operation, and to filter the fumes and smoke before returning the air to the room or blowing the air to an outside space.

Further improvements are needed, however, in fume extraction systems.

### SUMMARY

The present disclosure provides improvements to conventional fume extraction designs. Example systems can control fume suction from a welding tool during a welding operation, in particular, with a motor and/or filter carried by an operator, with the air filtered of fumes, particulates, and/or debris being released to the environment, a containment system, and/or to a welding helmet. These and other features and advantages of the present invention will be apparent from the following detailed description, in conjunction with the appended claims.

### BRIEF DESCRIPTION OF THE DRAWINGS

The benefits and advantages of the present invention will become more readily apparent to those of ordinary skill in the relevant art after reviewing the following detailed description and accompanying drawings, wherein:
FIG. 1 illustrates an example fume extraction system and associated welding torch, in accordance with aspects of this disclosure.
FIG. 2 illustrates another example fume extraction system and associated helmet, in accordance with aspects of this disclosure.
FIG. 3 illustrates another example fume extraction system with associated welding torch and helmet, in accordance with aspects of this disclosure.
FIGS. 4A through 4C illustrates an example motor for the fume extraction system of FIG. 4, in accordance with aspects of this disclosure.
FIG. 5 illustrates another example fume extraction system with a collection tank, in accordance with aspects of this disclosure.
FIG. 6 illustrates another example fume extraction system with associated welding torch and helmet, in accordance with aspects of this disclosure.
FIG. 7 illustrates schematic of airflow through various fume extraction systems, in accordance with aspects of this disclosure.

The figures are not necessarily to scale. Where appropriate, similar or identical reference numbers are used to refer to similar or identical components.

### DETAILED DESCRIPTION

Disclosed are systems and methods for fume extraction control. In particular, a fume extraction system, such as for a welding operation, includes one or more motors to control fume suction airflow. In some examples, a welding torch performs a welding operation on one or more workpieces, thereby creating fumes, debris, etc. In some examples, the fume extraction system is supported on the person of an operator, such as by a harness, belt, or straps, as a list of non-limiting examples. The system includes one or more motors to create suction, and one or more filters to collect fumes, particulates, and/or debris from the incoming air. The filtered air can then be exhausted and/or recycled, as disclosed in the examples provided herein. As a result, fumes and/or debris are efficiently and effectively collected and/or removed from the work environment.

Conventional fume extraction systems operate at a distance from the work environment, often restricting an operator's movements during welding. For example, an operator in need of fume extraction may have to conduct welding at a specific location within a facility that is equipped with fume mitigation systems.

For instance, fume extraction systems typically take one of three forms. One type of system is a large centralized system with fume arms or hoods, which may be expensive, stationary, and most useful for a shop with one or more fixed-site weld cells in need of fume extraction.

Another fume extraction system can have fume extraction arms and/or hoods, similar to a centralized system, but can be equipped with a power source, and be movable (e.g., mounted on wheels). The system is mobile and can be moved between different weld cells within a shop. However, even these mobile systems can be quite large, and may not be suited for small welding operations and/or field work.

Yet another type of fume extraction system is a source capture device integrated with a welding torch (i.e. fume gun). These systems may be attached to a metal inert gas (MIG) torch, with fume extraction nozzles and/or conduits built into and/or attached to the welding torch itself. The fume extraction conduits attached to the welding torch are connected to a suction source, e.g., an air suction system with a motor capable of high vacuum fume extraction.

Some shortcomings of these solutions include the need to plug into a substantial power source, are heavy, and require a large footprint. Even the most portable of these solution is still rather large and heavy (e.g., in excess of 40 lbs.). Conduit or hose lengths are also limited (e.g., 5-7 meters, less than 20 feet), and limits the user's mobility during a welding operation.

Operators may perform quick and/or less intense welding operations that need limited fume extraction or for a limited period of time. Disclosed fume extraction systems provide a solution that has a smaller, lighter, and more portable package, while providing air suction and filtering capability for a variety of welding operations. Such systems can be carried by the operator (e.g., via a belt, harness, strap, etc.), can be integrated with the welding torch (e.g., source capture), and can filter the fumes from incoming air at the source.

In disclosed examples, an amount of suction provided from a fume extraction system during a welding operation is controlled. Such control can be implemented remotely and "on the fly" during a welding operation and/or in response to changing welding operations. For example, welding operations may employ fume extraction source capture systems, which include fume nozzles and/or fume extraction conduits/hoses on or near a tip of the welding torch. The fume nozzles are connected to a high speed, high flow fume extraction unit. However, certain welding positions/applications operate most effectively with different amounts of suction. In order to avoid evacuation of needed shielding gases (and avoid possible weld porosity issues stemming therefrom), the fume extraction system can be controlled, as disclosed herein.

In an example, the amount of suction is remotely adjusted during welding by controlling a speed of a variable-frequency drive (VFD) motor that provides negative airflow into the fume extraction system from the fume nozzle. In some examples, valving or other suitable devices can be controlled to adjust the suction flow for a given welding torch. In particular, multiple welding torches (e.g., equipped with source capture devices) can be connected to a single fume extraction unit (e.g., via a single hose or multiple hoses). The valves/adjustment devices can be controlled individually to control the suction flow at the point of welding, independent of any other welding torch/welding site.

Control circuitry can be incorporated within the fume extraction system, on the welding torch itself, an associated robot welding system, and/or with another related machine (e.g., a wire feeder, welding power source, remote control). Suction flow may be controlled by a computing platform or control circuitry, such as in response to a monitored condition (e.g., via one or more sensors) and/or based on one or more parameters of an associated welding system (e.g., power output, wire feed speed, etc.). Sensor data of the monitored conditions may be used to determine if an adjustment in suction flow is needed, provide an alert to an operator as to the changing conditions, and/or control an adjustment to the system automatically. Control can be automatic (e.g., in response to one or more conditions monitored by sensors,) and/or in response to a user input (e.g., an input via a trigger, knob, pedal, touchscreen, etc.).

Advantageously, the disclosed fume extraction system is much smaller, making it ultra portable for short welding operations and for welds in hard to reach locations. Beneficially, the disclosed fume extraction system is fully contained, and does not require external hoses, power and/or connections. In some disclosed examples, the fume extraction system combines one or both of air filtration for a breathing apparatus (e.g., a powered air purifying respirator (PAPR)), and/or a wire feeder.

Furthermore, even when welding operations are being conducted out of doors, there is a benefit to filtering and collecting debris and particulates, thereby ensuring such contaminants are not returned to the environment.

According to aspects of this disclosure, example source capture fume evacuation systems include: an air inlet arranged proximate to a nozzle of a welding tool; and a body-worn suction device configured to draw in air from the air inlet, in which the suction device further includes: a motor driving a fan to create suction that draws in the air from the air inlet; a spark arrestor configured to deenergize sparks entrained in the air received from the air inlet; a filter to receive the air from the air inlet through the spark arrestor; and an air outlet to expel the filtered air.

In some example fume evacuation systems, the motor has a power of approximately 0.3 horsepower. In some example fume evacuation systems, the fan driven by the motor is capable of providing a volume flow rate of approximately 35 cubic feet per minute (CFM). In some example fume evacuation systems, the suction device draws in the air from the inlet through a conduit connecting the inlet to the suction device.

Some example fume evacuation systems further include an energy storage device configured to provide power to the motor. In some example fume evacuation systems, the suction device includes a belt or harness. In some example fume evacuation systems, the suction device includes a user interface including an input device to adjust a speed of the motor.

According to aspects of this disclosure, example fume evacuation systems include: an air inlet arranged proximate to a nozzle of a welding tool; a helmet configured to receive ambient air from a vent; and a suction device configured to draw in air from the air inlet and the vent, in which the suction device further includes: a first filter to receive the air from the air inlet; a second filter to receive the ambient air from the vent; an air outlet to expel the air from the air inlet; and a motor system configured to urge fumes from the air inlet to the air outlet via the first filter and to urge air from the vent to the helmet via the second filter.

In some example fume evacuation systems, the motor system includes at least one motor configured to drive a first impeller and a second impeller. In some example fume evacuation systems, the first impeller is geared higher than the second impeller, such that the at least one motor drives a single shaft to turn the first impeller at a first speed greater than a second speed of the second impeller. In some example fume evacuation systems, the first impeller pulls air through the first filter and a second impeller pulls air through the second filter.

In some example fume evacuation systems, the first impeller and second impeller pull air through the inlet air filter. In some example fume evacuation systems, the at least one motor includes first and second motors, the first motor to drive the first impeller and the second motor to drive the second impeller. In some example fume evacuation systems, the suction device is secured to a user by a belt or harness. In some example fume evacuation systems, the suction device further includes a blow-off valve, wherein extra air is evacuated from the suction device into the environment when a threshold pressure is exceeded. In some example fume evacuation systems, the suction device includes a user interface having an input device to adjust a speed of the motor. In some example fume evacuation systems, the suction device includes a spark arrestor configured to deenergize sparks entrained in the air received from the air inlet, and the first filter is configured to receive the air from the air inlet via the spark arrestor.

When introducing elements of various embodiments described below, the articles "a," "an," and "the" are intended to mean that there are one or more of the elements. The terms "comprising," "including," and "having" are intended to be inclusive and mean that there may be additional elements other than the listed elements. Moreover, while the term "exemplary" may be used herein in connection to certain examples of aspects or embodiments of the presently disclosed subject matter, it will be appreciated that these examples are illustrative in nature and that the term "exemplary" is not used herein to denote any preference or requirement with respect to a disclosed aspect or embodiment. Additionally, it should be understood that references to "one embodiment," "an embodiment," "some embodiments," and the like are not intended to be interpreted as excluding the existence of additional embodiments that also incorporate the disclosed features.

As used herein, the terms "coupled," "coupled to," and "coupled with," each mean a structural and/or electrical connection, whether attached, affixed, connected, joined, fastened, linked, and/or otherwise secured. As used herein, the term "attach" means to affix, couple, connect, join, fasten, link, and/or otherwise secure. As used herein, the term "connect" means to attach, affix, couple, join, fasten, link, and/or otherwise secure.

As used herein, the terms "first" and "second" may be used to enumerate different components or elements of the same type, and do not necessarily imply any particular order.

As used herein the terms "circuits" and "circuitry" refer to any analog and/or digital components, power and/or control elements, such as a microprocessor, digital signal processor (DSP), software, and the like, discrete and/or integrated components, or portions and/or combinations thereof, including physical electronic components (i.e., hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, circuitry is "operable" and/or "configured" to perform a function whenever the circuitry comprises the necessary hardware and/or code (if any is necessary) to perform the function, regardless of whether performance of the function is disabled or enabled (e.g., by a user-configurable setting, factory trim, etc.).

The terms "control circuit," "control circuitry," and/or "controller," as used herein, may include digital and/or analog circuitry, discrete and/or integrated circuitry, microprocessors, digital signal processors (DSPs), and/or other logic circuitry, and/or associated software, hardware, and/or firmware. Control circuits or control circuitry may be located on one or more circuit boards that form part or all of a controller.

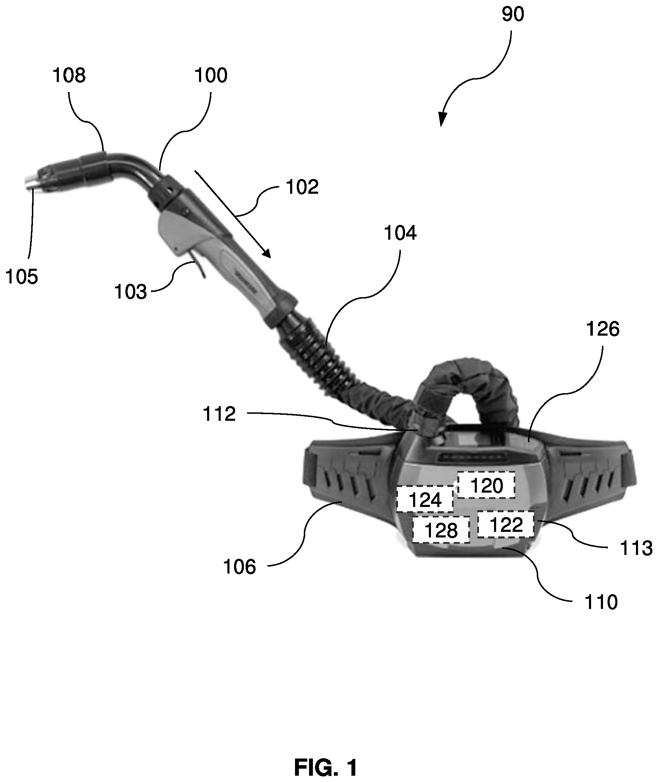

Turning now to the drawings, FIG. 1 illustrates a fume extraction system 90 for extracting fumes 102 and/or other airborne components, such as smoke, particulate matter, and more generally, workspace air from a work area. In the illustrated example, the fume extraction system 90 is incorporated with a welding torch 100. In the illustrated embodiment, the fume extraction system 90 includes one or more of a fume inlet 108, a conduit or hose 104, and a fume extraction unit 110. The fume inlet 108 is positioned proximate to a nozzle 105 of the welding torch 100. As shown, the conduit 104 is attached to the unit 110 at an inlet 112 to receive fumes 102. An outlet or vent 113 expels filtered/treated air from the unit 110.

The fume extraction unit 110 may include one or more of a motor 120 (e.g., a fixed speed motor, a variable speed motor, a VFD, etc.), filter(s) 122, and/or components 124 (e.g., control circuitry, a battery or other energy storage device, a flow adjustment device (e.g., valve, louver, etc.), a user interface 126 (e.g., switch, touchscreen, control panel, etc.), one or more sensors, and/or a motor 120). Various components (e.g., devices, circuits, motors, valves, louvers, actuators, alerts, etc.) of the welding system 90 can be controlled, engaged, activated, and/or manipulated to regulate airflow, such as through synchronization with the welding torch 100.

Further, the fume extraction unit 110 can include a belt, strap, and/or harness 106 to support the fume extraction system components, and aid in the operator's ability to carry the system with minimal disruption to mobility and performance during welding operations. In some examples, the hose 104 draws the fumes into a filter 122, which can be cleaned and/or collected by the filter 122. Once the fumes from the air have been treated, the air can be returned to the environment. Upon completion of the welding operation, the filter 122 can be removed and replaced.

In some examples, the filter 122 is designed to fit within the housing of the fume extraction unit 110, which would be considerably smaller than filters used in conventional systems. For instance, the filters be disposable, reusable, and/or recyclable. The filters can be layered, such that two or more filters of the same or different type (e.g., material type, porosity, shape, etc.) can be configured to filter airflow. The filters can be flat or curved, defined by one or more shapes (e.g., circular, square, cylindrical, ovoid, triangular, rectangular, etc.). In some examples, one or more conditions associated with the filter can be monitored to provide an indication of the filter serviceability. For instance, an airflow sensor may measure airflow rate, volume, etc., and determine if the filter needs replacement or cleaning. In some examples, the amount of current required to turn the motor provides an indication of airflow through the filter and therefore filter serviceability. An alert and/or icon can be presented to the operator once a determination is made that the filter requires attention to maintain a threshold amount of airflow.

The fumes are drawn through the filter 122 as the motor 120 (e.g., a 0.3 hp motor or smaller) creates suction (e.g., with a volume flow rate of approximately 35 cubic feet per minute (CFM)) through the fume inlet 108. Furthermore, as the length of conduit 104 is significantly shorter than conventional systems (e.g., approximately 1 meter, 3 ft), the motor power requirements are much lower, such that additional static friction or drag found when trying to pull air through length of a hose is greatly diminished. Thus, a small motor and filter can be employed, sized to fit within the housing of the unit 110, suitable for a belt mounted package.

In some disclosed examples, operation of the fume extraction system 90 can be synchronized or integrated with use of the welding torch 100. For example, activating the trigger 103 of the welding torch 100 can activate and/or adjust an amount of fume suction at the fume nozzle 108 (e.g., by controlling speed of the motor 120). For example, the user interface 126 may include one or more input devices (e.g., a dial, input buttons, etc.) to allow the operator to adjust the speed of the motor 120, to thereby adjust the fume collection.

Additionally or alternatively, a position of a flow adjustment device (e.g., a valve or louver), can be changed to achieve the desired suction airflow.

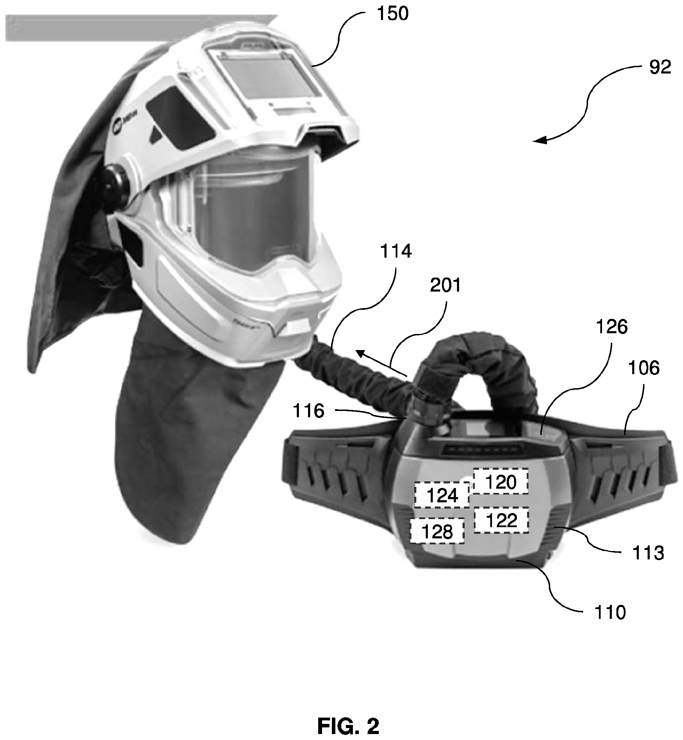

As shown in FIG. 2, one or more components of disclosed fume extraction systems 92 may be configured to operate with multiple types of welding torches and/or helmets 150. For example, the unit 110 may ingest fumes and/or environmental air (e.g., through inlet 112 shown in FIG. 1, and/or by reversing flow at exhaust 113), treat the air by the filter 122, and send treated air 201 to the helmet 150 via conduit 114. may provide positive-pressure air to and channel negative-pressure air and airborne components from the workspaces.

In some examples, the arrangement of system 92 employs the same components of the system 90, swapping the torch 100 with the helmet 150. In other words, a single hose is used, which can convey both a positive pressure (sending filtered air to the helmet 150) or outgoing flow or negative pressure (drawing in fumes from the torch 100). In some examples, the conduit 114 is additionally or alternatively connected to a wearable item or article of clothing configured to direct airflow to or toward a body part of the operator. For example, a cooling band or belt can be configured with holes, slits, etc., causing the air to blow against the operator, causing a cooling effect. Such cooling wearables/clothing can be used with any suction or airflow system disclosed herein.

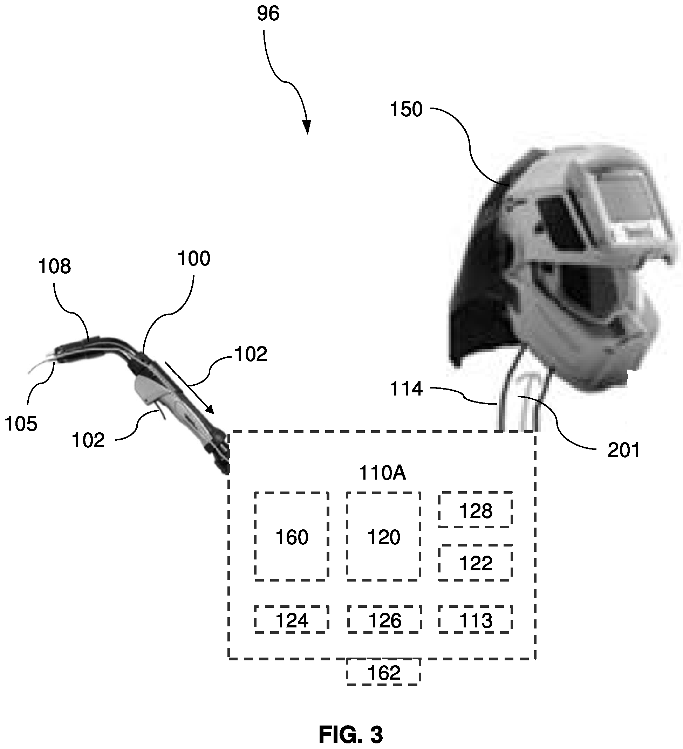

FIG. 3 illustrates another example fume extraction system 96 with the welding torch 100 and helmet 150, where modified unit 110A includes one or more of a motor 120, filter(s) 122, blow valve 162, and/or a wire feeder 160. As shown, the unit 110A can be attached to operator (e.g., via a belt, harness, strap, etc.) complete with wire feeder 160 and an fume evacuation system all in one housing, all attached as a backpack, for instance. In this manner, either a single power source and/or power line to a power source is used to power all available features. In examples, the amount of suction required to effectively employ the fume extraction feature is much higher than the airflow into the helmet. Thus, a blow valve 162 can be used to automatically and/or manually release extra pressure from the unit 110A as needed. In some examples, the torch is a spool gun with a dedicated wire spool/wire feeder, and also includes an incorporated fume extraction system.

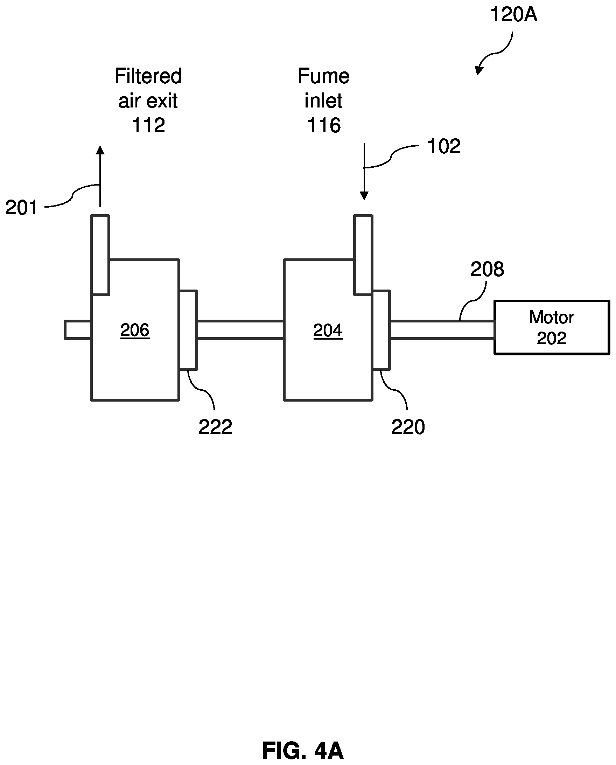

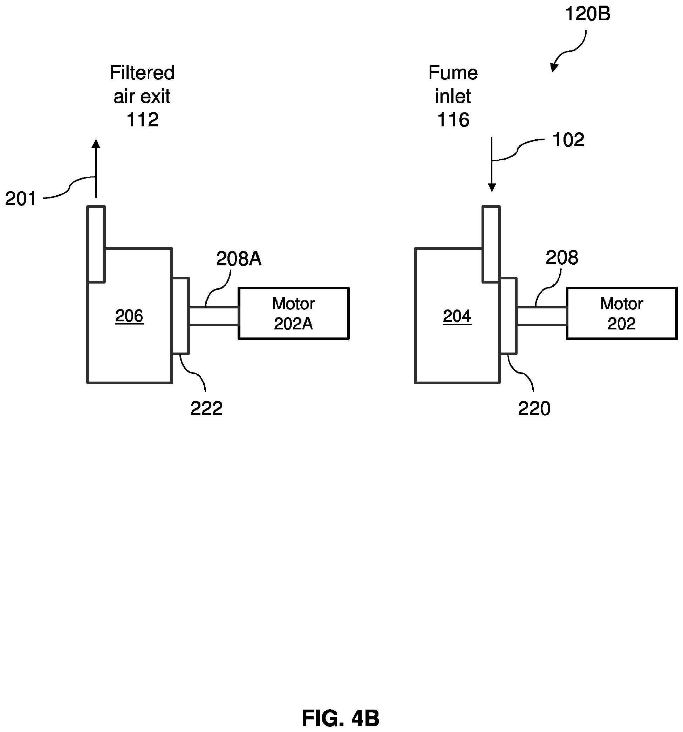

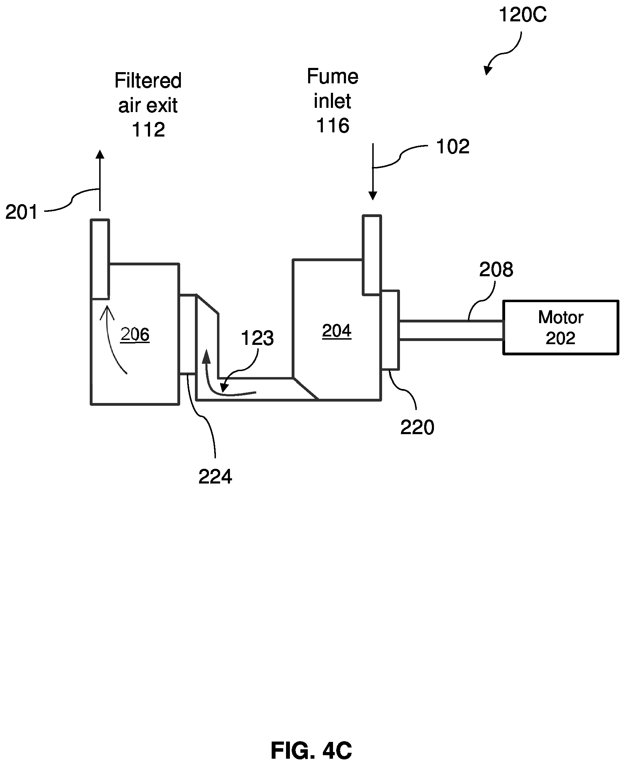

The system 96 (and/or system 94) can be configured to employ two motors (as shown in the example of FIG. 4B, one to draw fumes from the torch, and a second to provide air to the helmet), or employ a single motor suitable to operate in both modes simultaneously (as shown in the examples of FIGS. 4A and 4C). In an example shown in FIG. 4A, disclosed motor 120A include a dual impeller motor that has a first impeller 204 geared for the higher suction (e.g. CFM) needed for proper fume gun operation, and a second impeller 206 geared for lower suction to provide airflow to the helmet. The system could also employ a single filter 122 or multiple filters. For instance, a single filter can be designed to run both fume extraction and helmet airflow operations. In some examples, the system employs two filters: a first filter suitable for provision of air to the helmet; and a second filter suitable to treat fumes/particles from the welding torch. The first filter may a single filter or a combination of filters for effective filtration that meets standards for respiratory filtration (e.g., charcoal or High Efficiency Particulate Air (HEPA)). Such a filter may have a finer structure, to capture more and/or smaller particulates. The second filter may have a larger pore structure suitable for fume/debris collection.

The example systems 90, 92, 94, 96, 98 further include a spark arrestor 128 positioned between the fume inlet 108 and the filter 122. The spark arrestor 128 de-energizes sparks that may be entrained in the air flowing into the fume inlet 108, preventing high-energy sparks from reaching the filter 122. The spark arrestor 128 may include a tortuous path and/or baffles, a screen, a vortex chamber, and/or any other type of spark arrestor 128.

In the example system shown in FIG. 3, during dual mode operation, greater airflow/suction is needed to draw in fume at the welding torch, while substantially less airflow is needed to supply air to the helmet. One way to balance the pressure is to connect a blow off valve (e.g. valve 162) at a side of the housing/system that serves the fume extraction, so the volume, rate of airflow, suction level at the side supplying airflow to the helmet is at a desired level.

FIG. 3 shows a dual sided system, where the belt/backpack mounted system includes a spool of wire and a motor to drive the wire. Another side includes the fume evacuation motor and filter(s) that could treat fumes from a welding torch, supply filtered air to the helmet, or perform both operations simultaneously. The blow off valve is a feature employed if a single impeller and/or motor is used. Because of the different airflow requirements of the fume gun and the helmet, a blow off valve is used to dissipate extra air to release pressure. For instance, if the fume extraction has a suction level of 30 cfm, while the helmet has a desired suction level of 5 cfm, the blow off valve can regulate the air provided to the helmet and release air into the environment to lower the suction level by approximately 25 cfm.

In some examples, as shown in FIG. 4A, a dual impeller 120A fan is used to provide the different airflow suction rates. A motor 202 turns a shaft 208 to drive a first impeller 204 and a second impeller 206. At the first impeller 204, fume 102 is drawn into the system, while at the second impeller 206 filtered air 212 is sent to the helmet. The two impellers 204, 206 may have different gearing, such that they rotate at different speeds, resulting in different amounts of suction from each that is suitable for their desired application. For example, gearing 220 can be configured to rotate impeller 204 at a first speed, such that the amount of suction from the fume source (e.g., the fume nozzle 108) draws in a first volume of air 102 at a first flow rate. Gearing 222 is configured to rotate impeller 206 at a second speed (slower than the first speed), such that the second volume of air 201 being expelled is a second volume and a second flow rate (e.g., less than the first volume and/or rate). In some other examples, the impellers 204, 206 are driven at a same speed. In some examples, the impellers 204, 206 are differently sized to provide different airflow volumes and/or different airflow speeds at the same impeller speed and/or different impeller speeds (via different gearing).

In some examples, the both impellers 204 and 206 may be activated simultaneously, while in other examples one or the other impeller may be selectively driven by the shaft 208. The selection can be implemented via electronic control (e.g., one or more switches or levers actuated via commands from control circuitry) and/or via mechanical control (e.g., one or more manual switches or levers). Selective control can enable the system 120B to operate in one or more modes: source capture mode; PAPR mode; and dual impeller mode. In some examples, a blow off valve may or may not be employed. With dual impellers providing suction at different amounts and levels, a blow off valve may not be necessary, but may be desirable for balancing pressure within the system.

As shown in FIG. 4B, motor 120B includes two motors. A first motor 202 can be used to turn a first fan or impeller 204, whereas second motor 202A can be used to turn a second fan or impeller 206. The motors 202 and 202A may have different power requirements and/or outputs, as the vacuum requirements (e.g., airflow, volume and/or pressure) for fume extraction is greater than that for respiratory applications.

In yet another example motor system 120C shown in FIG. 4C, the two impellers can be driven by a single motor 202 in a turbo-like configuration. For instance, a motor 202 is sufficient to supply CFMs suitable for the fume extraction, with air 123 generated from the first impeller 204 delivered and/or diverted to drive the second impeller 206. The air 123 may be concentrated to drive a turbo fan, impeller, gearing, etc., to turn the impeller 206 to provide the airflow 201. This can be switched automatically and/or manually, such that one or both modes can be in use during any given operation.

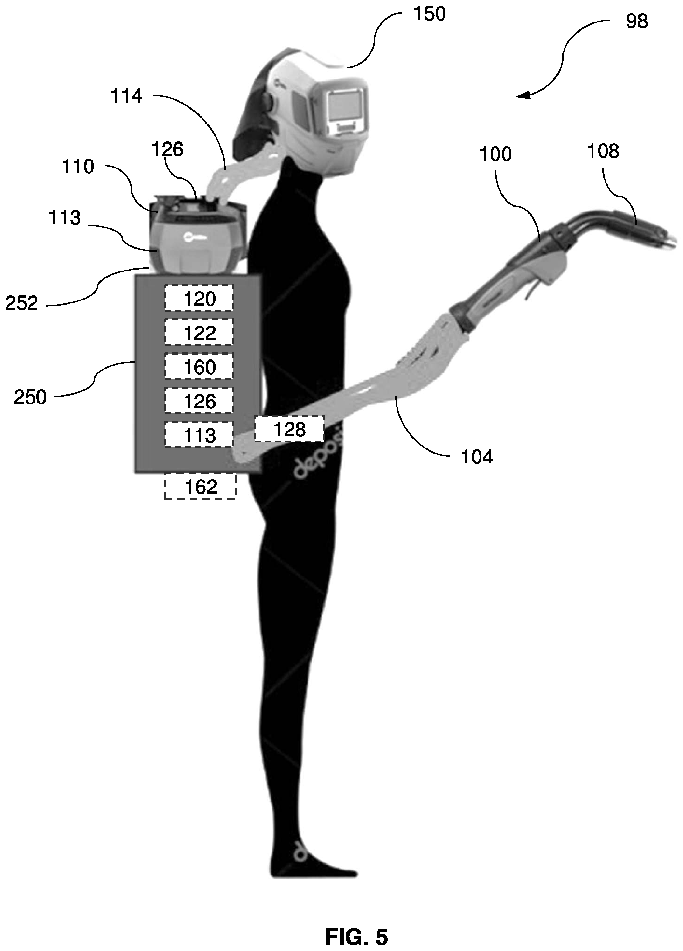

FIG. 5 illustrates another example fume extraction system with a collection tank 250. In some examples, the fume extraction unit 98 could be configured to operate in a fume extraction mode alone. In such an example, the collection tank 250 can be integrated with other components, such as a wire feeder (e.g., 160), a motor (e.g., 120A), and one or more filters. A standalone system may also include the components of unit 110, including circuitry and a power source.

In some examples, the system 98 can further be configured to receive a unit 110 to filter air and provide the filtered air to the helmet 150. For instance, the unit 110 may be configured to mate with a mount or fastener 252, which may secure the unit 110 to the container 250. In some examples, the mount 252 further integrates the unit 110 with the fume evacuation system 98, such that power and/or control is provided to the unit 110 from the system 98. This can allow for synchronization between units, and may allow for the unit 110 to forego inclusion of a battery, as power from the system 98 comes from either a larger battery, or connected to mains power. In some examples, the unit 110 remains fully independent, such that its functioning (e.g., filtered air to the helmet 150) does not require power/control from the system 98.

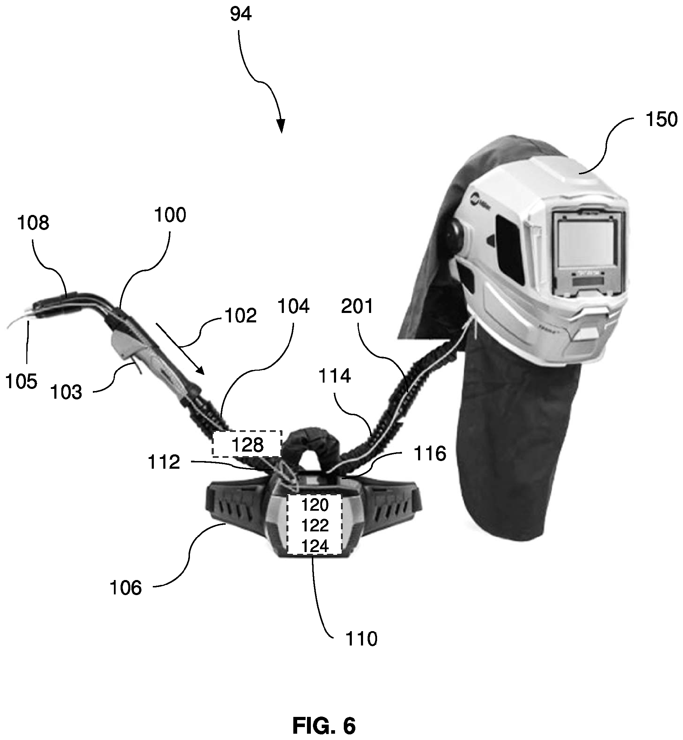

FIG. 6 illustrates another example fume extraction system 94 connected to both the welding torch 100 and the helmet 150. In the disclosed example, the system 94 is capable of running in multiple distinct modes. One mode is defined as a source capture mode, using a higher suction power, thereby pulling in air and fume through the fume nozzle 108. The fumes/air are filtered and expelled to the environment. Another breathing PAPR mode draws in air from the environment, filters the air, and provides filtered air 201 into the helmet 150. In breathing mode, less suction power (and thus less CFM) is required, thus fans/motors can operate at a lower speed. Selection between modes can be activated by selection on the user interface 126.

In some examples, the unit 110 would be configured to operate in a dual function mode, such that the source capture and breathing modes can work simultaneously. In some examples, fumes are drawn in through hose 104, filtered (e.g., by a first filter) and exhausted into the environment. At the same time, air is drawn in from the environment, filtered (e.g., by a second filter), and provided to the helmet 150 via hose 114. Such an arrangement ensures there is no cross-fume contamination. Beneficially, during use in cold weather, heated air from the unit 110 will be sent to helmet 150.

In additional or alternative examples, air/fumes pulled into the fume nozzle 108 can be filtered, and then pushed into the helmet of the user. However, when a shielding gas is being used, a separate oxygen source may be needed to provide oxygen to the helmet. For example, shielding gas can be made of inert gases that could include argon, carbon dioxide, helium, or some other gas other than oxygen. To avoid pushing an inert gas into the helmet, a physical gas separator can be included to prevent contamination. In some examples, enhanced filtering systems can include micro-fractional distillation, pressure swing absorption membranes (PSA), and/or a vacuum pressure swing absorption (VPSA) can be used to prevent shielding gas from being introduced into the helmet.

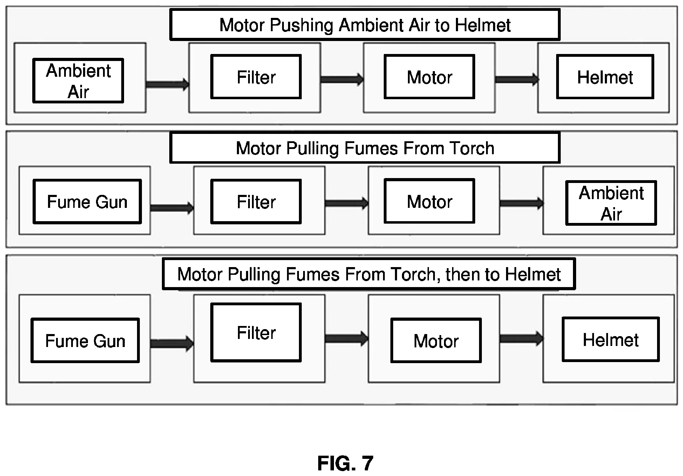

FIG. 7 illustrates a schematic of airflow through various arrangements of various disclosed fume extraction systems. In practice, air pathways through the systems are kept separate to ensure proper filtering.

In a first instance, the motor draws in ambient air from the environment, which is filtered prior to being provided to the helmet (see, e.g., FIG. 2). In a second instance, the motor draws in fumes from the torch, which is filtered prior to being evacuated to the environment (see, e.g., FIG. 1). In a third instance, the motor draws in fumes from the torch, which is filtered prior to being provided to the helmet (see, e.g., FIG. 3).

In some examples, original equipment or even retrofits for the disclosed fume extraction systems may be made to equipment such as shop vacuum systems, existing evacuation installations, and so forth. It is also contemplated that structures and teachings based on those set forth herein may be employed in specific settings to provide enhanced fume and airborne component collection.

As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations.

While the present method and/or system has been described with reference to certain implementations, it will be understood by those skilled in the art that various changes may be made and equivalents may be substituted without departing from the scope of the present method and/or system. In addition, many modifications may be made to adapt a particular situation or material to the teachings of the present disclosure without departing from its scope. For example, systems, blocks, and/or other components of disclosed examples may be combined, divided, re-arranged, and/or otherwise modified. Therefore, the present method and/or system are not limited to the particular implementations disclosed. Instead, the present method and/or system will include all implementations falling within the scope of the appended claims, both literally and under the doctrine of equivalents.

Various embodiments of the invention are specified in the following clauses.

Clause 1. A source capture fume evacuation system comprising:
an air inlet arranged proximate to a nozzle of a welding tool; and
a body-worn suction device configured to draw in air from the air inlet, wherein the suction device further comprises:
   a motor driving a fan to create suction that draws in the air from the air inlet;
   a spark arrestor configured to deenergize sparks entrained in the air received from the air inlet;
   a filter to receive the air from the air inlet through the spark arrestor; and
   an air outlet to expel the filtered air.

Clause 2. The fume evacuation system of clause 1, wherein the motor has a power of approximately 0.3 horsepower.

Clause 3. The fume evacuation system of clause 2, wherein the fan driven by the motor is capable of providing a volume flow rate of approximately 35 cubic feet per minute (CFM).

Clause 4. The fume evacuation system of clause 3, wherein the suction device draws in the air from the inlet through a conduit connecting the inlet to the suction device.

Clause 5. The fume evacuation system of clause 1, further comprising an energy storage device configured to provide power to the motor.

Clause 6. The fume evacuation system of clause 1, wherein the suction device comprises a belt or harness.

Clause 7. The fume evacuation system of clause 1, wherein the suction device comprises a user interface including an input device to adjust a speed of the motor.

Clause 8. A fume evacuation system comprising:
an air inlet arranged proximate to a nozzle of a welding tool;
a helmet configured to receive ambient air from a vent; and
a suction device configured to draw in air from the air inlet and the vent, wherein the suction device further comprises:
   a first filter to receive the air from the air inlet;
   a second filter to receive the ambient air from the vent;
   an air outlet to expel the air from the air inlet; and
   a motor system configured to urge fumes from the air inlet to the air outlet via the first filter and to urge air from the vent to the helmet via the second filter.

Clause 9. The fume evacuation system of clause 8, wherein the motor system includes at least one motor configured to drive a first impeller and a second impeller.

Clause 10. The fume evacuation system of clause 9, wherein the first impeller is geared higher than the second impeller, such that the at least one motor drives a single shaft to turn the first impeller at a first speed greater than a second speed of the second impeller, and optionally the first impeller pulls air through the first filter and a second impeller pulls air through the second filter.

Clause 11. The fume evacuation system of clause 9, wherein the at least one motor includes first and second motors, the first motor to drive the first impeller and the second motor to drive the second impeller.

Clause 12. The fume evacuation system of clause 8, wherein the suction device is secured to a user by a belt or harness.

Clause 13. The fume evacuation system of clause 8, wherein the suction device further comprises a blow-off valve, wherein extra air is evacuated from the suction device into the environment when a threshold pressure is exceeded.

Clause 14. The fume evacuation system of clause 8, wherein the suction device comprises a user interface including an input device to adjust a speed of the motor.

Clause 15. The fume evacuation system of clause 8, wherein the suction device comprises a spark arrestor configured to deenergize sparks entrained in the air received from the air inlet, and the first filter is configured to receive the air from the air inlet via the spark arrestor.

## Claims

1. A source capture fume evacuation system comprising:
an air inlet arranged proximate to a nozzle of a welding tool; and
a body-worn suction device configured to draw in air from the air inlet, wherein the suction device further comprises:
a motor driving a fan to create suction that draws in the air from the air inlet;
a spark arrestor configured to deenergize sparks entrained in the air received from the air inlet;
a filter to receive the air from the air inlet through the spark arrestor; and
an air outlet to expel the filtered air.

2. The fume evacuation system of claim 1, wherein the motor has a power of approximately 0.3 horsepower.

3. The fume evacuation system of claim 2, wherein the fan driven by the motor is capable of providing a volume flow rate of approximately 35 cubic feet per minute (CFM).

4. The fume evacuation system of claim 3, wherein the suction device draws in the air from the inlet through a conduit connecting the inlet to the suction device.

5. The fume evacuation system of claim 1, further comprising an energy storage device configured to provide power to the motor.

6. The fume evacuation system of claim 1, wherein the suction device comprises a belt or harness.

7. The fume evacuation system of claim 1, wherein the suction device comprises a user interface including an input device to adjust a speed of the motor.

8. A fume evacuation system comprising:
an air inlet arranged proximate to a nozzle of a welding tool;
a helmet configured to receive ambient air from a vent; and
a suction device configured to draw in air from the air inlet and the vent, wherein the suction device further comprises:
a first filter to receive the air from the air inlet;
a second filter to receive the ambient air from the vent;
an air outlet to expel the air from the air inlet; and
a motor system configured to urge fumes from the air inlet to the air outlet via the first filter and to urge air from the vent to the helmet via the second filter.

9. The fume evacuation system of claim 8, wherein the motor system includes at least one motor configured to drive a first impeller and a second impeller.

10. The fume evacuation system of claim 9, wherein the first impeller is geared higher than the second impeller, such that the at least one motor drives a single shaft to turn the first impeller at a first speed greater than a second speed of the second impeller, and optionally the first impeller pulls air through the first filter and a second impeller pulls air through the second filter.

11. The fume evacuation system of claim 9, wherein the at least one motor includes first and second motors, the first motor to drive the first impeller and the second motor to drive the second impeller.

12. The fume evacuation system of claim 8, wherein the suction device is secured to a user by a belt or harness.

13. The fume evacuation system of claim 8, wherein the suction device further comprises a blow-off valve, wherein extra air is evacuated from the suction device into the environment when a threshold pressure is exceeded.

14. The fume evacuation system of claim 8, wherein the suction device comprises a user interface including an input device to adjust a speed of the motor.

15. The fume evacuation system of claim 8, wherein the suction device comprises a spark arrestor configured to deenergize sparks entrained in the air received from the air inlet, and the first filter is configured to receive the air from the air inlet via the spark arrestor.
